(19) Europäisches Patentamt / European Patent Office / Office européen des brevets

(11) **EP 2 707 093 B1**

(12) **FASCICULE DE BREVET EUROPEEN**

(45) Date de publication et mention de la délivrance du brevet:
**06.03.2019 Bulletin 2019/10**

(21) Numéro de dépôt: **12728663.1**

(22) Date de dépôt: **11.05.2012**

(51) Int Cl.:
*A61N 1/36* (2006.01)    *A61F 9/08* (2006.01)
*G06K 9/46* (2006.01)    *G09B 21/00* (2006.01)
*G06F 19/00* (2018.01)    *G06N 3/04* (2006.01)
*A61N 1/05* (2006.01)

(86) Numéro de dépôt international:
**PCT/FR2012/051052**

(87) Numéro de publication internationale:
**WO 2012/153073 (15.11.2012 Gazette 2012/46)**

(54) **PROCEDE ET DISPOSITIF DE COMMANDE D'UN DISPOSITIF D'AIDE A LA VISION**

VERFAHREN UND VORRICHTUNG ZUR STEUERUNG EINES VORRICHTUNG ZUR SEHHILFE

METHOD AND DEVICE FOR CONTROLLING A DEVICE FOR AIDING VISION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **12.05.2011 FR 1154116**

(43) Date de publication de la demande:
**19.03.2014 Bulletin 2014/12**

(73) Titulaires:
• **SORBONNE UNIVERSITE**
 **75006 Paris (FR)**
• **Centre National de la Recherche Scientifique (C.N.R.S.)**
 **75794 Paris Cedex 16 (FR)**

(72) Inventeurs:
• **LORACH, Henri**
 **F-75005 Paris (FR)**
• **BENOSMAN, Ryad**
 **F-75016 Paris (FR)**
• **SAHEL, José**
 **F-75013 Paris (FR)**
• **PICAUD, Serge**
 **F-77210 Avon (FR)**

(74) Mandataire: **Cabinet Plasseraud**
 **66, rue de la Chaussée d'Antin**
 **75440 Paris Cedex 09 (FR)**

(56) Documents cités:
 **US-A1- 2007 299 484    US-A1- 2009 112 287**

**US-A1- 2010 036 457**

• LIU S C ET AL: "Neuromorphic sensory systems", CURRENT OPINION IN NEUROBIOLOGY, LONDON, GB, vol. 20, no. 3, 1 juin 2010 (2010-06-01), pages 288-295, XP027079056, ISSN: 0959-4388, DOI: 10.1016/J.CONB.2010.03.007 [extrait le 2010-05-20]
• LI-JU LIN ET AL: "A Neuromorphic Chip That Imitates the ON Brisk Transient Ganglion Cell Set in the Retinas of Rabbits", IEEE SENSORS JOURNAL, IEEE SERVICE CENTER, NEW YORK, NY, US, vol. 7, no. 9, 1 septembre 2007 (2007-09-01), pages 1248-1261, XP011187666, ISSN: 1530-437X, DOI: 10.1109/JSEN.2007.901194
• ANDREAS THIEL ET AL: "The temporal structure of transient ON/OFF ganglion cell responses and its relation to intra-retinal processing", JOURNAL OF COMPUTATIONAL NEUROSCIENCE, KLUWER ACADEMIC PUBLISHERS, BO, vol. 21, no. 2, 26 mai 2006 (2006-05-26), pages 131-151, XP019398617, ISSN: 1573-6873, DOI: 10.1007/S10827-006-7863-X
• BOAHEN K ET AL: "Optic Nerve Signals in a Neuromorphic Chip I: Outer and Inner Retina Models", IEEE TRANSACTIONS ON BIOMEDICAL ENGINEERING, IEEE SERVICE CENTER, PISCATAWAY, NJ, USA, vol. 51, no. 4, 1 avril 2004 (2004-04-01), pages 657-666, XP011109270, ISSN: 0018-9294, DOI: 10.1109/TBME.2003.821039

- MARKUS SCHWARZ ET AL: "Single-Chip CMOS Image Sensors for a Retina Implant System", IEEE TRANSACTIONS ON CIRCUITS AND SYSTEMS II: ANALOG AND DIGITALSIGNAL PROCESSING/ISSN 1057-7130>VN 8090B, INSTITUTE OF ELECTRICAL AND ELECTRONICS ENGINEERS INC, 345 EAST 47 STREET, NEW YORK, N.Y. 10017, USA, vol. 46, no. 7, 1 juillet 1999 (1999-07-01), XP011013088, ISSN: 1057-7130
- PATRICK LICHTSTEINER ET AL: "A 128128 120 dB 15 s Latency Asynchronous Temporal Contrast Vision Sensor", IEEE JOURNAL OF SOLID-STATE CIRCUITS, IEEE SERVICE CENTER, PISCATAWAY, NJ, USA, vol. 43, no. 2, 1 February 2008 (2008-02-01), pages 566-576, XP011200748, ISSN: 0018-9200, DOI: 10.1109/JSSC.2007.914337
- A Acosta-Jiménez ET AL: "AER Building Blocks for Multi-Layer Multi-Chip Neuromorphic Vision Systems", Neural Information Processing Systems, 1 December 2005 (2005-12-01), XP055464354, Vancouver Retrieved from the Internet: URL:http://www.ini.ethz.ch/~tobi/caviar/ca viarPosterNIPS2005.pdf [retrieved on 2018-04-03]
- R. Serrano-Gotarredona ET AL: "AER Building Blocks for Multi-Layer Multi-Chip Neuromorphic Vision Systems", Advances in neural information processing systems 18, 1 January 2006 (2006-01-01), pages 1217-1224, XP055464358, Retrieved from the Internet: URL:https://papers.nips.cc/paper/2889-aer-building-blocks-for-multi-layer-multi-chip -neuromorphic-vision-systems.pdf [retrieved on 2018-04-03]

**Description**

La présente invention concerne le domaine des dispositifs d'aide à la vision, et plus particulièrement la manière de commander des prothèses ou orthèses pour fournir des informations visuelles aux personnes qui les portent.

La réhabilitation de la vue par des prothèses visuelles vise à stimuler des neurones le long du chemin visuel entre la rétine et le cerveau, pour évoquer une perception signifiante chez des personnes aveugles. L'implant d'une prothèse visuelle est destiné à venir au voisinage de cellules nerveuses et à appliquer un champ électrique modulé spatialement et temporellement. Le champ électrique est appliqué localement dans des zones de pixel organisées en matrice. Il induit des potentiels électriques sur les membranes des neurones qui reçoivent son influence. Des cellules encore fonctionnelles de la rétine ou du cortex visuel peuvent ainsi être stimulées alors même que les photorécepteurs ou d'autres cellules de la rétine ne sont plus fonctionnels.

Des approches existantes prennent pour cibles différents emplacements du système visuel. Les implants sous-rétiniens stimulent des cellules bipolaires de la rétine, tandis que les implants épi-rétiniens stimulent des cellules ganglionnaires qui sont connectées au cerveau par l'intermédiaire du nerf optique. Dans ces deux stratégies, on cherche à utiliser les cellules restantes de la rétine après dégénérescence des cellules photoréceptrices. Une autre approche utilise des implants corticaux qui stimulent directement le cortex visuel et peuvent donc être utilisés y compris dans des cas où le nerf optique est endommagé. Ces trois stratégies ont été testées dans des essais cliniques et ont montré qu'elles pouvaient évoquer des phosphènes et permettre la reconnaissance de formes et même dans certains cas la reconnaissance de lettres.

Les orthèses ont pour objectif de présenter des informations visuelles prétraitées et donc simplifiées sur des zones de rétine encore fonctionnelles pour apporter des informations visuelles manquantes. Ces informations peuvent être manquantes car en regard d'un scotome ou inaccessibles normalement de par leur complexité, leur taille ou leur contraste (vision augmentée).

Les dispositifs d'aide à la vision (prothèses ou orthèses) sont alimentées avec des signaux obtenus en traitant ceux fournis par un système d'acquisition de lumière. Des stratégies classiques consistent à effectuer une acquisition lumineuse sous forme d'images ou de trames vidéo régulièrement espacées dans le temps. Cette méthode d'échantillonnage, utilisée par exemple dans US 2010/0067825 A1, soulève plusieurs difficultés.

Un traitement de l'image souvent lourd, comme l'extraction de la salience ou l'extraction des contours, est appliqué aux images afin de définir un schéma d'activation du dispositif d'aide à la vision. Actuellement, les différentes stratégies de stimulation choisies n'ont pas donné de résultats satisfaisants. Les limites de cette méthode proviennent de la faible dynamique du capteur fournissant au mieux une image toutes les 33 ms. D'autre part, la mise en place de caméras à couplage de charge (CCD, "charge-coupled device") plus rapides est incompatible avec la complexité des algorithmes de traitement d'image et ne convient pas pour un système portatif.

La reproduction de caractéristiques dynamiques du système visuel requiert en effet des temps de réponse très courts. Il a été montré chez le mammifère que le cerveau parvient à extraire certaines caractéristiques du champ visuel dans des délais de quelques dizaines de millisecondes seulement. Le retard de traitement imputable à la rétine est de l'ordre de 50 ms. Quand un échantillonnage image par image est effectué, il est nécessaire d'accumuler quelques images pour observer des gradients temporels porteurs d'information. Le temps de 50 ms pour la modélisation de la rétine est déjà dépassé si on accumule deux images à 40 Hz. Par conséquent, une extraction de caractéristiques précise et en temps réel par la rétine nécessite théoriquement une fréquence d'échantillonnage supérieure à 60 Hz pour calculer des dérivées temporelles du second ordre, traiter le signal et en extraire des caractéristiques.

En outre, les stimuli élémentaires doivent être positionnés dans le temps avec une précision de quelques millisecondes en raison de la dynamique très rapide de la chaîne de traitement des informations visuelles (voir "Rapid Neural Coding in the Retina with Relative Spike Latencies", T. Gollisch, et al., Science, Vol. 319, février 2008, pp. 1108-1111). Cette exigence ne peut pas être remplie avec les systèmes d'acquisition image par image ayant des fréquences d'échantillonnage réalistes. Les publications suivantes représentent également l'état de la technique dans le domaine des dispositif d'aide à la vision:

-   Acosta-Jiménez et al.: "AER Building Blocks for Multi-Layer Multi-Chip Neuromorphic Vision Systems", Neural Information Processing Systems, décembre 2005, URL:http://www.ini.ethz.ch/~tobi/caviar/caviarPosterNIPS2005.pdf
-   R. Serrano-Gotarredona et al.: "AER Building Blocks for Multi-Layer Multi-Chip Neuromorphic Vision Systems", Advances in neural information processing systems 18, 2005, URL:https://papers.nips.cc/paper/2889-aer-building-blocks-for-multi-layer-multi-chip-neuromorphic-vision-systems.pdf

Il existe donc un besoin pour des techniques permettant de commander de manière appropriée des dispositifs d'aide à la vision.

Il est proposé un dispositif de traitement de signal pour la commande d'un dispositif d'aide à la vision tel que défini par la revendication 1. Les revendications dépendantes définissent des modes de réalisation de la présente invention.

Les aspects, modes de réalisation, exemples ou procédés décrits dans la présente description qui ne sont pas couverts par les revendications ne font pas partie de l'invention.

L'utilisation de signaux asynchrones pour construire les signaux de commande du dispositif d'aide à la vision présente de nombreux avantages. Ces signaux ne sont pas échantillonnés dans le temps selon une fréquence d'horloge prédéfinie, comme l'horloge des trames dans un signal vidéo classique. Ils fournissent ce qu'on appelle une représentation adresse-événement (AER, "address-event représentation") d'une scène à visualiser. À chaque pixel correspond une séquence de signal basé sur événement ("event-based"), c'est-à-dire dépendant des variations d'intensité lumineuse correspondant à ce pixel. Dans un exemple de réalisation, la séquence de signal asynchrone basé sur événement pour un pixel comporte une séquence d'impulsions positives ou négatives positionnées dans le temps en fonction des variations de lumière concernant le pixel. Ce type d'acquisition permet de reproduire le mode de d'acquisition lumineuse continue des photorécepteurs de la rétine. Il tire parti du degré élevé de redondance temporelle dans le champ de vision. Ainsi:

- on n'a pas besoin de répéter dans le temps les niveaux lumineux sensiblement constants vus par la majorité des pixels, comme le fait une caméra classique ayant une fréquence de trames donnée;

- on est capable de rendre compte avec rapidité et précision du positionnement dans le temps de variations lumineuses locales, sans être limité par une période inter-trames.

Les séquences de signal asynchrone subissent une transformation dans l'espace et le temps pour fournir les informations utiles pour les orthèses ou les prothèses visuelles. Plusieurs approches peuvent être adoptées pour cette transformation. En général, il sera nécessaire adapter la commande, et donc les paramètres de la transformation du signal, aux besoins du porteur.

Une approche possible est basée sur un modèle de comportement des différents types de cellules de la rétine.

La transformation du signal d'entrée pour générer les signaux de commande peut notamment comporter:

- obtenir un premier signal résultant de deux opérations de filtrage spatial avec des noyaux de filtrage de tailles différentes, du calcul d'une différence entre les résultats des deux opérations de filtrage spatial et d'une opération de filtrage temporel de la différence; et

- obtenir un second signal de valeur nulle si le premier signal a une valeur d'un signe déterminé, et de même valeur absolue que le premier signal sinon.

Les noyaux de filtrage de tailles différentes peuvent être vus comme rendant compte du comportement des photorécepteurs de la rétine d'une part et des cellules horizontales d'autre part, ces dernières ayant typiquement un rayon d'interaction plus important que celui des photorécepteurs. Le second signal reproduisant la partie positive ou négative du premier signal peut être vu comme étant celui engendré par une cellule bipolaire. La polarité de la différence calculée permet de distinguer des cellules bipolaires 'ON' et des cellules bipolaires 'OFF'. Différents jeux de paramètres de filtrage spatial et/ou temporel permettent en outre de distinguer les comportements de cellules bipolaires de différents types, étant donné qu'on sait qu'il existe au moins dix types différents de cellules bipolaires.

Ce genre de transformation peut convenir pour des prothèses visuelles en position sous-rétinienne, les signaux de commande appliqués à la prothèse visuelle étant alors générés à partir du second signal. Elle peut également convenir pour une orthèse comportant une matrice d'éléments photo-émetteurs.

Il est également possible de poursuivre la transformation au-delà de l'obtention des seconds signaux susmentionnés. Dans une réalisation, au moins un premier signal d'excitation et un premier signal d'inhibition sont obtenus avec des constantes de temps respectives pour l'opération de filtrage temporel de la différence, puis au moins un second signal d'excitation et un second signal d'inhibition sont respectivement obtenus à partir des premiers signaux d'excitation et d'inhibition. Les canaux excitateurs et inhibiteurs ainsi simulés correspondent à des cellules bipolaires qui peuvent fournir l'entrée excitatrice d'une cellule ganglionnaire et son entrée inhibitrice par l'intermédiaire de cellules amacrines. La transformation du signal d'entrée pour générer les signaux de commande comporte alors, après l'obtention de ces seconds signaux:

- obtenir un troisième signal résultant d'une opération de filtrage spatial de la différence entre le second signal d'excitation et une composante d'inhibition dérivée du second signal d'inhibition; et

- lorsque le troisième signal pour une zone de pixel donnée du dispositif d'aide à la vision dépasse une valeur de seuil déterminée, insérer une impulsion dans le signal de commande destiné à ladite zone de pixel et remettre à zéro le troisième signal pour ladite zone de pixel.

Dans le modèle, la dérivation de la composante d'inhibition à partir du second signal d'inhibition est imputable à des cellules amacrines, et peut comprendre l'application d'un retard prédéterminé et une opération de filtrage spatial.
Un signal de commande généré à partir d'un troisième signal obtenu de cette manière peut, pour certains patients, convenir pour une prothèse visuelle implantée en position épi-rétinienne ou corticale ou sur le corps genouillé latéral.
Une possibilité intéressante, permettant de reproduire le comportement d'une cellule ganglionnaire sensible à la direction, est d'utiliser un noyau de filtrage excentré dans l'opération de filtrage spatial intervenant dans la dérivation de la composante d'inhibition. Le décalage spatial du noyau de filtrage, combiné au retard induit par les cellules amacrines, donne lieu à une sensibilité de la réponse à la direction de mouvement des stimuli.
Certaines cellules ganglionnaires peuvent être excitées de manière combinée à partir de cellules bipolaires de types différents. Pour en rendre compte, on peut obtenir des seconds signaux d'excitation et d'inhibition d'une part pour un premier canal et d'autre part pour un second canal avec des opérations de filtrage temporel à constantes de temps respectives. La transformation du signal d'entrée pour générer les signaux de commande comporte alors, après l'obtention de ces seconds signaux:

- obtenir un troisième signal résultant d'une opération de filtrage spatial de la différence entre une combinaison linéaire des seconds signaux d'excitation pour les premier et second canaux et une composante d'inhibition dérivée des seconds signaux d'inhibition pour les premier et second canaux; et

- lorsque le troisième signal pour une zone de pixel donnée de la prothèse visuelle dépasse une valeur de seuil déterminée, insérer une impulsion dans le signal de commande destiné à cette zone de pixel et remettre à zéro le troisième signal pour la zone de pixel.

Dans le modèle, la dérivation de la composante d'inhibition à partir des seconds signaux d'inhibition est imputable à des cellules amacrines d'un type différent de celui mentionné précédemment, et peut comprendre l'application de retards respectifs aux seconds signaux d'inhibition pour les premier et second canaux, une opération de filtrage spatial des seconds signaux d'inhibition retardés et le calcul d'une combinaison linéaire des seconds signaux d'inhibition retardés et filtrés.
Un signal de commande généré à partir d'un troisième signal obtenu de cette manière peut, pour certains patients, convenir pour une prothèse visuelle implantée en position épi-rétinienne ou corticale ou sur le corps genouillé latéral. Elle peut également convenir pour une orthèse comportant une matrice d'éléments photo-émetteurs.
Des modèles différents, plus ou moins basés sur le comportement connu des cellules nerveuses, peuvent servir de référence pour la mise au point de la transformation particulière qui sera appliquée aux signaux de commande de la prothèse d'un patient donné. Des tests psychophysiques pourront être mis en oeuvre pour retenir finalement la transformation la plus appropriée à une personne donnée.
Il est encore envisageable de mettre au point cette transformation sans référence à un modèle phénoménologique, par exemple en utilisant un réseau de neurones artificiel.
D'autres particularités et avantages de la présente invention apparaîtront dans la description ci-après d'exemples de réalisation non limitatifs, en référence aux dessins annexés, dans lesquels :

- la figure 1 est un schéma synoptique d'un exemple d'équipement de stimulation du système visuel d'un patient souffrant d'une déficience de la vue;

- la figure 2A est un diagramme montrant un exemple de profil d'intensité lumineuse au niveau d'un pixel d'un capteur asynchrone;

- la figure 2B montre un exemple de signal délivré par le capteur asynchrone en réponse au profil intensité de la figure 2A;

- la figure 2C illustre la reconstruction du profil intensité à partir du signal de la figure 2B;

- les figures 3A-B sont des diagrammes analogues à ceux des figures 2A-B illustrant un mode d'acquisition lumineuse utilisable dans un autre exemple de réalisation du procédé;

- la figure 4 est une représentation schématique de différentes catégories de cellules nerveuses de la rétine;

- la figure 5 est un schéma illustrant des réponses de plusieurs types cellulaires de la rétine selon un modèle; et

- les figures 6 à 9 sont des schémas indiquant des traitements utilisables dans plusieurs exemples de réalisation du procédé.

Le rôle de la rétine est d'encoder le flux lumineux qu'elle reçoit en une séquence de potentiels d'action transmise au cerveau par l'intermédiaire du nerf optique. La cascade de la photo-transduction ainsi que les interactions entre différents types cellulaires au sein de la rétine entraînent un schéma complexe d'activation des cellules ganglionnaires. Ainsi, des estimations prédisent quelques dizaines de types de réponses des cellules ganglionnaires en fonction de leur morphologie et de leur physiologie.

Malgré cette variété dans les types de réponses observées, il a été démontré qu'une précision temporelle de l'ordre de quelques millisecondes dans la séquence de potentiels d'action est essentielle à une bonne interprétation de cette information par le cerveau. Il est utile de tenter de reproduire fidèlement la dynamique des cellules de la rétine lorsque l'on envisage un traitement prosthétique de la cécité. Le principe du traitement est la stimulation électrique des cellules de la rétine dans des cas de maladies dégénératives des photorécepteurs.

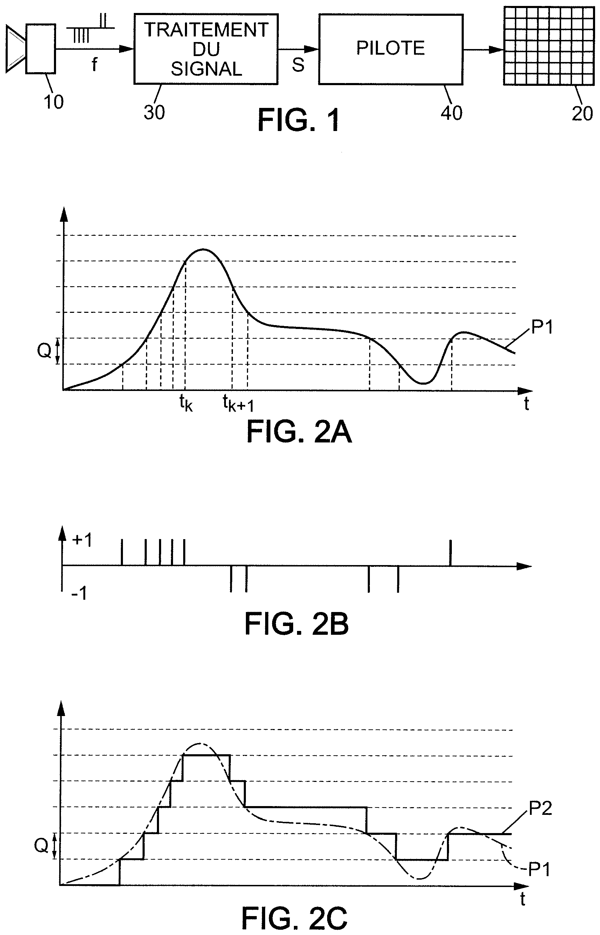

L'équipement utilisé (figure 1) comporte, dans cette application, un appareil d'acquisition de lumière 10 ayant un groupement d'éléments photosensibles organisés en une matrice de pixels et une prothèse 20 installée par exemple sur la rétine. Une implantation corticale de la prothèse 20 est également envisageable. Une unité de traitement 30 transforme le signal d'entrée f issu de l'appareil d'acquisition de lumière 10 en un ensemble de signaux de commande S destinés à des zones de pixel respectives de la prothèse 20. Pour appliquer ces signaux de commande S à la prothèse 20, ils sont convertis en des potentiels électriques analogiques par une unité de pilotage 40 qui transmet ces potentiels aux électrodes de la prothèse.

À titre d'exemple, la prothèse 20 peut-être du type décrit dans la demande de brevet FR 10 53381 déposée le 30 avril 2010. Ses zones de pixel incluent chacune une paire d'électrodes pour l'application locale d'une différence de potentiel qui stimule les cellules nerveuses soumises au champ électrique ainsi induit. L'une des deux électrodes peut appartenir à un plan de masse commun à une partie au moins des zones de pixel. Les zones de pixel de la prothèse 20 ont une densité spatiale qui n'a pas besoin de correspondre à la résolution spatiale de la matrice de pixels de l'appareil d'acquisition de lumière 10.

L'unité de traitement 30 qui fournit les signaux de commande S fonctionne sur des signaux numériques. Elle peut être implémentée par programmation d'un processeur approprié. Dans la pratique, une implémentation matérielle de l'unité de traitement de signal 30 à l'aide de circuits logiques spécialisés pourra être préférée pour une industrialisation de l'équipement.

Pour chaque pixel de la matrice, l'appareil 10 engendre une séquence de signal asynchrone basé sur événement à partir des variations de lumière ressenties par le pixel dans la scène apparaissant dans le champ de vision de l'appareil. Ce type d'appareil photosensible asynchrone permet d'approcher la réponse physiologique de la rétine et donc de produire un schéma de commande approprié. On le désigne ci-après par l'acronyme DVS : "Dynamic Vision Sensor" (capteur de vision dynamique).

Le principe d'acquisition par ce capteur asynchrone est illustré par les figures 2A-C. L'information consiste en une succession de temps $t_k$ (k = 0, 1, 2, ...) auxquels un seuil d'activation Q est atteint. La figure 2A montre un exemple de profil d'intensité lumineuse P1 vue par un pixel de la matrice du DVS. Chaque fois que cette intensité augmente d'une quantité égale au seuil d'activation Q à partir de ce qu'elle était au temps $t_k$, un nouvel instant $t_{k+1}$ est identifié et une raie positive (niveau +1 sur la figure 2B) est émise à cet instant $t_{k+1}$. Symétriquement, chaque fois que l'intensité du pixel diminue de la quantité Q à partir de ce qu'elle était au temps $t_{k'}$, un nouvel instant $t_{k'+1}$ est identifié et une raie négative (niveau -1 sur la figure 2B) est émise à cet instant $t_{k'+1}$. La séquence de signal asynchrone pour le pixel consiste alors en une succession d'impulsions ou raies positives ou négatives positionnées dans le temps aux instants $t_k$ dépendant du profil lumineux pour le pixel. Ces raies peuvent être représentées mathématiquement par des pics de Dirac positifs ou négatifs et caractérisées chacune par un instant d'émission $t_k$ et un bit de signe. La sortie du DVS 10 est ainsi sous la forme d'une représentation adresse-événement (AER). La figure 2C montre le profil d'intensité P2 qu'on est capable de reconstruire comme une approximation du profil P1 par intégration dans le temps du signal asynchrone de la figure 2B.

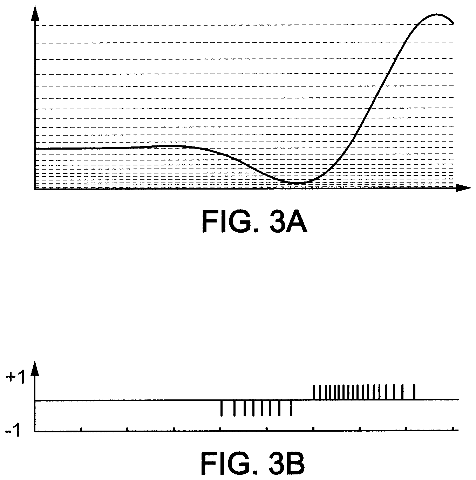

Le seuil d'activation Q peut être fixe, comme dans le cas des figures 2A-C, ou adaptatif en fonction de l'intensité lumineuse, comme dans le cas des figures 3A-B. Par exemple, le seuil $\pm Q$ peut être comparé aux variations du logarithme de l'intensité lumineuse pour la génération d'un événement $\pm 1$.

À titre d'exemple, le DVS 10 peut être du genre décrit dans "A 128×128 120 dB 15 $\mu$s Latency Asynchronous Temporal Contrast Vision Sensor", P. Lichtsteiner, et al., IEEE Journal of Solid-State Circuits, Vol. 43, No. 2, février 2008, pp. 566-576, ou dans la demande de brevet US 2008/0135731 A1.

La dynamique de la rétine (durée minimum entre les potentiels d'action) de l'ordre de quelques millisecondes peut être convenablement reproduite avec un DVS de ce type. La performance en dynamique est en tout cas largement supérieure à celle qu'on peut atteindre avec une caméra vidéo classique ayant une fréquence d'échantillonnage réaliste.

Il est à noter que la forme du signal asynchrone délivré pour un pixel par le DVS 10, qui constitue le signal d'entrée de l'unité de traitement 30, peut être différente d'une succession de pics de Dirac, les événements représentés pouvant avoir une largeur temporelle ou une amplitude ou une forme d'onde quelconque dans ce signal asynchrone basé sur événement.

D'autre part, le signal d'entrée n'est pas obligatoirement issu d'un appareil de détection de lumière. Il peut aussi être un signal AER synthétisé.

Afin de stimuler efficacement des cellules de la rétine, il convient non seulement d'avoir une dynamique d'acquisition suffisante mais également d'être capable de traiter le signal d'acquisition de manière pertinente. Chaque type de cellule du système visuel possède son propre schéma d'activation. À titre d'exemple, certaines cellules ganglionnaires répondent préférentiellement à une orientation donnée, à un mouvement, ou à un contraste. Ces propriétés émergent de la connectivité du réseau rétinien. Dans le cas des prothèses épi-rétiniennes, il convient de reproduire cette connectivité afin d'obtenir un timing de stimulation pertinent.

Une approche est d'entraîner un réseau de neurones artificiel à partir de données physiologiques, pour relier l'activité de chaque type de cellule ganglionnaire avec le signal du DVS. Le signal en provenance des différents pixels du DVS est introduit dans un réseau de neurones qui intègre les entrées pour fournir une prédiction de l'activité de la cellule ganglionnaire. À l'aide d'algorithmes connus, les poids intervenant dans les connexions du réseau artificiel sont ajustés jusqu'à la convergence de la prédiction vers une activité réelle mesurée. La précision temporelle obtenue grâce à ce principe d'acquisition et de filtrage permet de produire une stimulation asynchrone de la rétine pertinente du point de vue physiologique.

Une autre approche est de faire référence à un modèle de comportement des cellules nerveuses de la rétine dans la conception du traitement du signal effectué par l'unité 30.

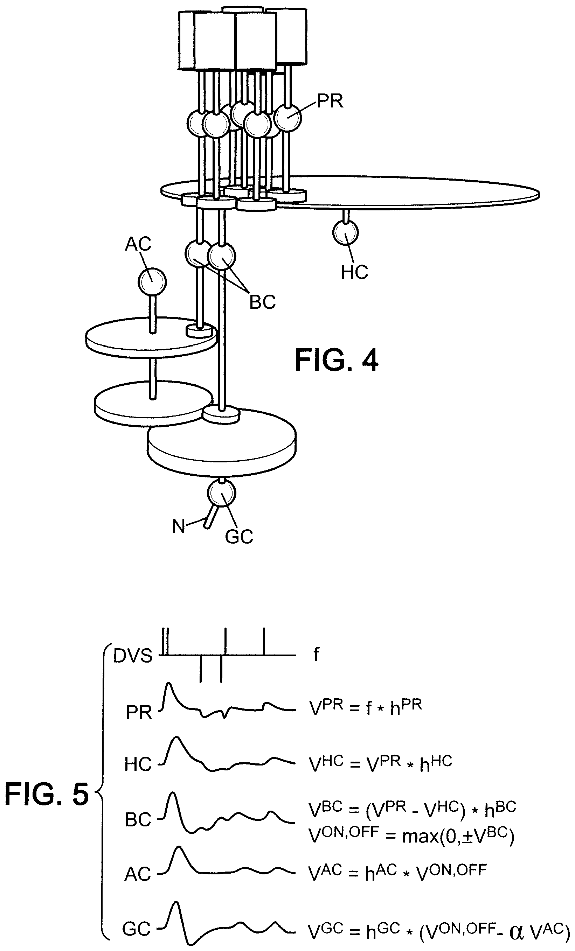

Le modèle peut être basé sur une structure générale de la rétine telle que celle représentée schématiquement sur la figure 4. Dans ce modèle, des convolutions spatiales et/ou temporelles sont effectuées à chaque niveau cellulaire. Les cellules bipolaires (BC, "bipolar cells") effectuent une transduction non linéaire soit de la partie positive soit de la partie négative du signal issu des photorécepteurs (PR) après inhibition retardée de la part des cellules horizontales

(HC, "horizontal cells"). Certaines cellules bipolaires activent des canaux 'ON' en réponse à des stimuli positifs, tandis que d'autres activent des canaux 'OFF' en réponse à des stimuli négatifs. Les cellules amacrines (AC, "amacrine cells") peuvent introduire des interactions entre les canaux 'ON' et/ou 'OFF' avec une inhibition retardée. Cette inhibition peut également introduire des gradients spatiaux dans le cas d'une inhibition décalée. Les cellules ganglionnaires (GC, "ganglion cells") reçoivent une excitation en provenance des cellules bipolaires des canaux 'ON' et/ou 'OFF' et une inhibition en provenance des cellules amacrines, et se comportent comme des neurones effectuant une intégration à fuites et une émission de potentiels d'action ("spikes") vers le nerf optique N.

Ces traitements sont résumés sur la figure 5. On peut considérer qu'un type cellulaire effectue une convolution d'un signal d'entrée $V = V(x, y, t)$ à l'aide d'un noyau de convolution $h = h(x, y, t)$ ayant une composante spatiale, par exemple de forme gaussienne avec un écart-type représentant un rayon $r$ d'interaction des cellules, et une composante temporelle avec une constante de temps $\tau$. Une forme possible du noyau de convolution est ainsi:

$$h(x, y, t) = \frac{t-D}{\tau^2}.\exp\left[-\frac{t-D}{\tau}\right].\frac{1}{2\pi r^2}.\exp\left[-\frac{(x-x_0)^2+(y-y_0)^2}{2r^2}\right] \qquad (1)$$

où: $x, y$ désignent les positions le long des deux directions spatiales;

$t$ désigne le temps;

$D$ désigne un paramètre de retard pouvant intervenir ($D \neq 0$) pour quelques types de cellules, en particulier des cellules amacrines;

$x_0, y_0$ désignent des paramètres de décalage spatial pouvant intervenir ($x_0 \neq 0$ et/ou $y_0 \neq 0$) pour quelques types de cellules, en particulier des cellules amacrines.

Sur la figure 5, la première ligne représente la séquence d'impulsions positives et négatives constituant le signal $f = f(x, y, t)$ issu du DVS 10 pour un pixel de position $(x, y)$ dans la matrice d'entrée. Le traitement par les photorécepteurs consiste en l'application du noyau de convolution $h^{PR}$ au signal d'entrée $f$. Le traitement par les cellules horizontales consiste en l'application du noyau de convolution $h^{HC}$ au signal $V^{PR}$ issus des photorécepteurs pour former un signal $V^{HC}$. Les cellules bipolaires appliquent le noyau de convolution $h^{BC}$ à la différence $(V^{PR}-V^{HC})$ pour former un signal $V^{BC}$ dont seule la partie positive est conservée dans le cas d'un canal 'ON' ($V^{ON} = \max\{0, V^{BC}\}$) et seule la partie négative est conservée dans le cas d'un canal 'OFF' ($V^{OFF} = \max\{0, -V^{BC}\}$). Les noyaux $h^{PR}, h^{HC}, h^{BC}$ ont des paramètres $\tau, r$ différenciés. Pour différents types de cellules bipolaires à modéliser, on prévoit différents jeux de paramètres $\tau, r$

dont les valeurs peuvent être déterminées séparément.

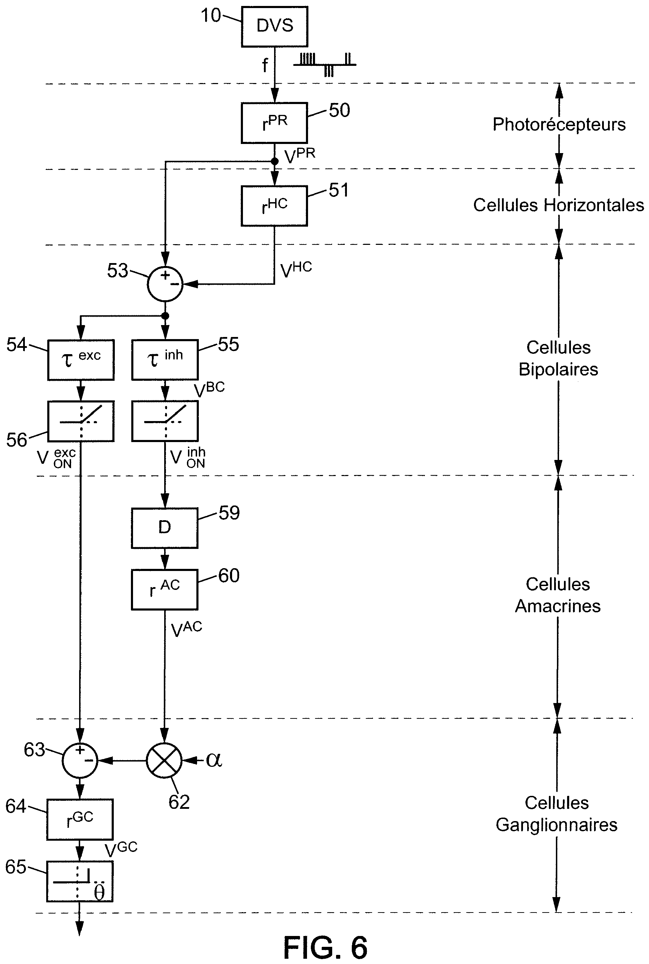

Compte tenu de la linéarité des opérations effectuées jusqu'à la modélisation des cellules bipolaires, il est possible, dans l'exemple représenté sur la figure 6, de n'effectuer que des convolutions spatiales 50, 51 au titre des photorécepteurs et des cellules horizontales (noyaux $h^{PR}$, $h^{HC}$ ayant des rayons d'interaction $r^{PR}$, $r^{HC}$) et une convolution simplement temporelle 54 ou 55 au titre d'un type de cellules bipolaires (noyaux $h^{BC}$ ayant des constantes de temps $\tau^{exc}$ pour une cellule excitatrice et $\tau^{inh}$ pour une cellule inhibitrice). Dans ce modèle, les rayons $r^{PR}$, $r^{HC}$ peuvent rendre compte d'intégrations spatiales qui, en réalité, se répartissent le long de la chaîne comportant les trois types cellulaires. De même, les constantes de temps $\tau^{exc}$, $\tau^{inh}$ peuvent rendre compte d'intégrations temporelles qui, en réalité, se répartissent le long de la chaîne comportant les trois types cellulaires, voire en outre des cellules amacrines et des cellules ganglionnaires. Sur la figure 6, le soustracteur 53 calcule la différence entre les signaux $V^{PR}$ et $V^{HC}$ pour qu'elle soit filtrée temporellement, et les éléments 56, 57 conservent la partie positive du signal $V^{BC}$ pour fournir la sortie simulée excitatrice ou inhibitrice, $V^{ON} = V^{exc}_{ON}$ ou $V^{inh}_{ON}$, d'une cellule bipolaire 'ON'.

La figure 6 montre également un exemple de modélisation du comportement des cellules amacrines et des cellules ganglionnaires. Dans cet exemple, la couche de cellules amacrines reçoit le signal inhibiteur $V^{inh}_{ON}$ provenant d'une cellule bipolaire 'ON', le retarde d'un temps D (unité de retard 59), et le filtre spatialement avec un noyau de convolution 60 de rayon $r^{AC}$ (par exemple un noyau centré, c'est-à-dire avec $x_0 = y_0 = 0$). Le signal résultant est une composante d'inhibition $V^{AC}$ adressée à la couche de cellules ganglionnaires avec le signal excitateur $V^{exc}_{ON}$ provenant d'une cellule bipolaire qui, dans l'exemple de la figure 6, est également une cellule 'ON'. La couche de cellules ganglionnaires d'un type donné est modélisée par un multiplieur 62 qui pondère la composante d'inhibition $V^{AC}$ par un coefficient $\alpha$, un soustracteur 63 qui retranche la composante d'inhibition pondérée $\alpha.V^{AC}$ du signal d'excitation $V^{exc}_{ON}$, un filtre 64 qui applique le noyau de convolution $h^{GC}$, qui se limite ici à un noyau spatial de rayon $r^{GC}$, et un émetteur de potentiels d'action 65 recevant le signal convolué $V^{GC} = h^{GC}{}_*(V^{exc}_{ON} - \alpha.V^{AC})$. Chaque fois que le signal de tension $V^{GC}$ atteint un seuil positif $\theta$, l'émetteur 65 produit un potentiel d'action ("spike") sur son signal de sortie et remet à zéro le signal de tension $V^{GC}$, réinitialisant ainsi le processus d'intégration intervenant dans le filtrage 64.

Un modèle tel que celui représenté sur la figure 6 permet de reproduire les réponses de certaines cellules ganglionnaires 'ON'. À partir de mesures de potentiels d'action observés sur le nerf optique et/ou de courants excitateurs et inhibiteurs en réponse à un stimulus donné (voir B. Roska, et al., "Parallel Processing in Retinal Ganglion Cells: How Integration of Space-Time Patterns of Excitation and Inhibition Form the Spiking Output", Journal of Neurophysiology, Vol. 95, No. 6, 2006, pp. 3810-3822), un processus d'optimisation permet de sélectionner les paramètres du modèle les mieux adaptés pour reproduire au mieux les observations. Dans le cas de la figure 6, les paramètres en question sont $r^{PR}$, $r^{HC}$, $\tau^{exc}$, $\tau^{inh}$, D, $r^{AC}$, $\alpha$, $r^{GC}$ et $\theta$.

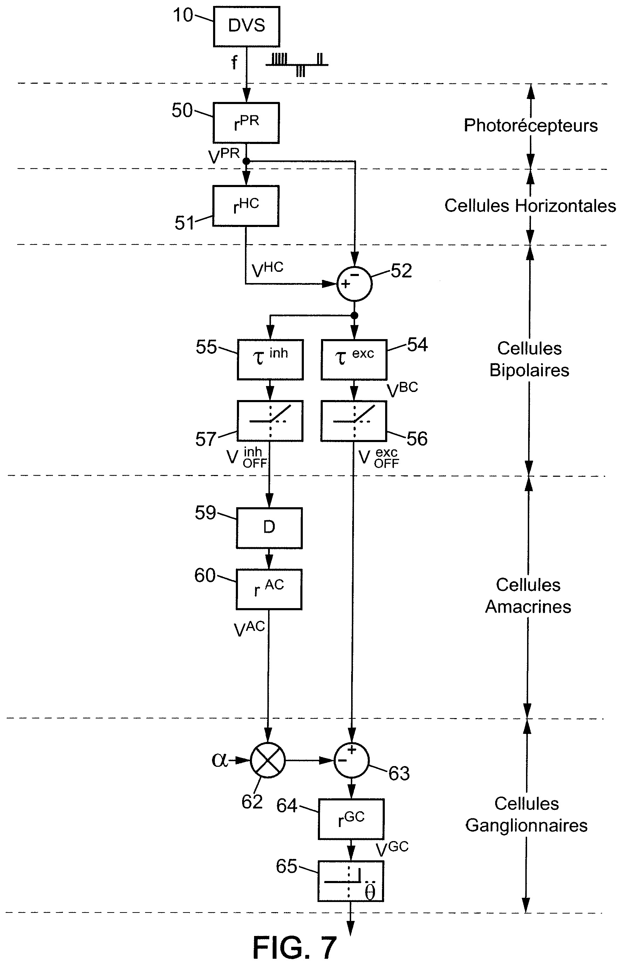

Le cas de la figure 6 est celui de cellules ganglionnaires 'ON', c'est-à-dire réagissant à des stimuli sous forme de raies positives du signal d'entrée. Le modèle est aisément transposable au cas de cellules ganglionnaires 'OFF', c'est-à-dire réagissant à des stimuli sous forme de raies négatives du signal d'entrée. Le schéma peut alors être celui de la figure 7, qui est le même que celui de la figure 6, à cette différence près que la soustraction 52 entre les signaux $V^{PR}$ et $V^{HC}$ intervenant dans la couche des cellules bipolaires a sa polarité inversée par rapport à celle 53 de la figure 6.

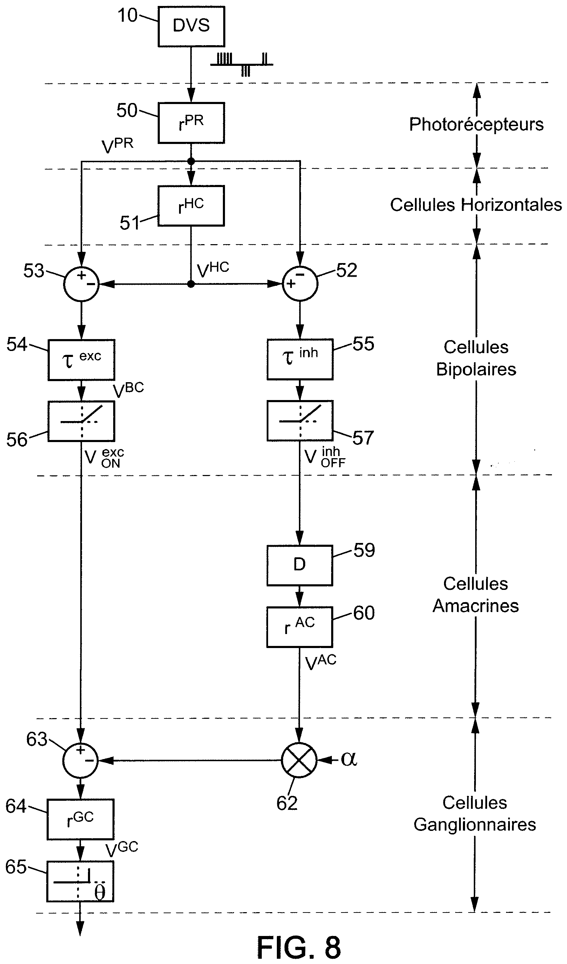

Une autre situation possible est que des cellules ganglionnaires reçoivent leurs signaux d'excitation $V^{exc}_{ON}$ (ou $V^{exc}_{OFF}$) de cellules bipolaires 'ON' (ou 'OFF') alors que leurs composantes d'inhibition $V^{AC}$ sont obtenues à partir de cellules bipolaires 'OFF' (ou 'ON'). Cette situation est illustrée par la figure 8 dans un cas d'excitation par des cellules bipolaires 'ON' et d'inhibition par des cellules bipolaires 'OFF'.

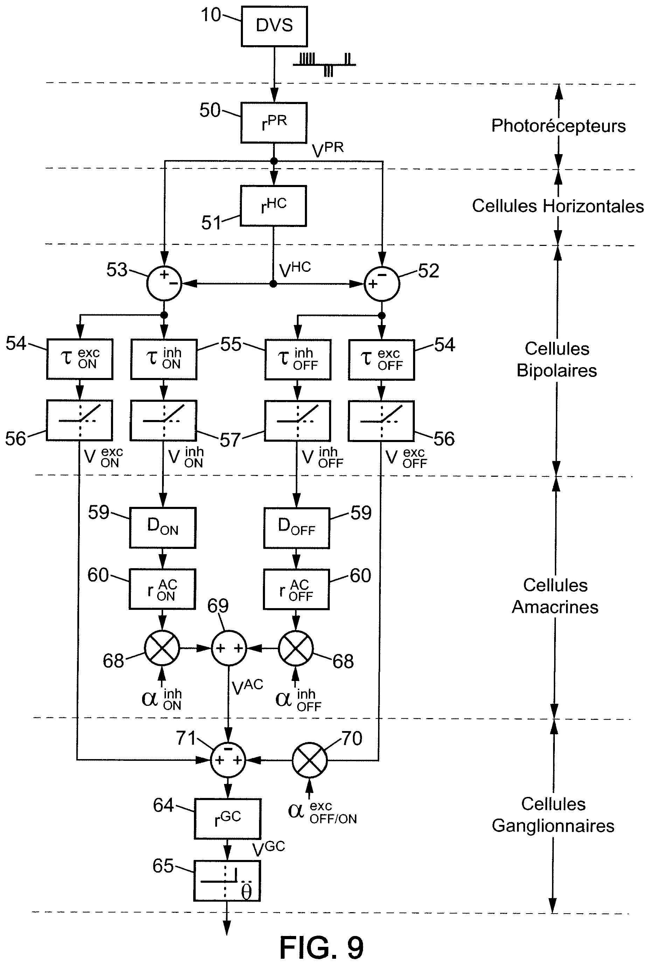

Une autre possibilité encore, illustrée par la figure 9, est que des cellules ganglionnaires soient excitées à la fois par des cellules bipolaires 'ON' et 'OFF' (avec une pondération relative réglée par un coefficient positif $\alpha^{exc}_{OFF/ON}$), et inhibées par des composantes $V^{AC}$ issues de combinaisons de signaux d'inhibition $V^{inh}_{ON}$, $V^{inh}_{OFF}$ émis par des cellules bipolaires 'ON' et 'OFF'. Deux familles de cellules amacrines sont alors modélisées dans un tel canal, avec des temps différents $D_{ON}$, $D_{OFF}$ au niveau des unités de retard 59 et éventuellement des rayons d'interaction différents $r^{AC}_{ON}$, $r^{AC}_{OFF}$ au niveau des filtres 60. Les sorties des deux filtres 60 sont combinées linéairement pour former la composante d'inhibition $V^{AC}$. Dans l'exemple de la figure 9, la combinaison est réalisée par des multiplieurs 68, qui appliquent des coefficients de pondération respectifs $\alpha_{ON}^{inh}$, $\alpha_{OFF}^{inh}$ aux signaux filtrés de la voie canal 'ON' et de la voie 'OFF', et un additionneur 69 qui produit la composante d'inhibition $V^{AC}$ comme la somme des signaux filtrés et pondérés.

Dans la modélisation de la couche de cellules ganglionnaires selon la figure 9, un multiplieur 70 applique le coefficient de pondération $\alpha_{OFF/ON}^{exc}$ au signal d'excitation $V_{OFF}^{exc}$ issu de la cellule bipolaire 'OFF'. Les composantes d'excitation et d'inhibition sont combinées en 71 pour fournir l'entrée $V_{ON}^{exc} + \alpha_{OFF/ON}^{exc} \cdot V_{OFF}^{exc} - V^{AC}$ du filtre spatial 64. Les valeurs des coefficients de pondération $\alpha_{OFF/ON}^{exc}$, $\alpha_{ON}^{inh}$, $\alpha_{OFF}^{inh}$ permettent de régler les niveaux relatifs d'excitation et d'inhibition en provenance des différentes cellules bipolaires impliquées.

Dans une variante du schéma de la figure 9, l'entrée du filtre spatial 64 n'est pas $V_{ON}^{exc} + \alpha_{OFF/ON}^{exc} \cdot V_{OFF}^{exc} - V^{AC}$ mais $\alpha_{ON/OFF}^{exc} \cdot V_{ON}^{exc} + V_{OFF}^{exc} - V^{AC}$. Le coefficient $\alpha_{OFF/ON}^{exc}$ ou $\alpha_{ON/OFF}^{exc}$ est positif ou nul. On peut le contraindre à être nul, auquel cas les cellules ganglionnaires sont excitées par un seul type de cellules bipolaires et inhibées à partir de deux types de cellules bipolaires via des cellules amacrines.

Pour des cellules ganglionnaires relevant d'autres canaux d'information, on peut encore ajouter au modèle d'autres schémas d'excitation mettant en jeu des paramètres différenciés.

À partir du signal AER issu du capteur DVS 10, une modélisation telle qu'illustrée par les figures 5 à 9 a permis de reproduire convenablement, par optimisation des paramètres du modèle, les dix types de réponse décrits chez des rongeurs dans l'article précité de B. Roska, et al. Ceci montre la capacité du procédé à fournir une stimulation pertinente des cellules nerveuses de la rétine.

Pour des cellules ganglionnaires sensibles à la direction, le modèle peut être enrichi pour inclure un décalage $x_0$, $y_0$ dans le noyau de filtrage spatial 60 intervenant dans la couche représentant le traitement incombant aux cellules amacrines. Ce noyau excentré, combiné au retard D appliqué par ces cellules amacrines, rend compte d'une directivité des stimuli suivant l'orientation du décalage $x_0$, $y_0$.

Lorsque la prothèse est implantée en position épi-rétinienne, elle influence le potentiel électrique de cellules ganglionnaires. Le signal de commande S délivré par l'unité de traitement du signal 30 de la figure 1 peut alors être celui produit par l'émetteur de potentiels d'action 65 représenté sur l'une des figures 6 à 9. Le type de cellules ganglionnaires à stimuler et le schéma de modélisation correspondant peuvent être sélectionnés pour un patient donné en lui faisant suivre des tests psychophysiques pour rechercher le mode de commande fournissant les meilleurs résultats perceptuels. Éventuellement, certains des paramètres du modèle peuvent en outre être réglés lors de tels tests. Une autre implantation possible de la prothèse est sur le cortex et le corps genouillé latéral (c'est-à-dire l'intermédiaire entre la rétine et le cortex). Dans ce dernier cas, on peut par exemple appliquer à la prothèse des signaux semblables à ceux mentionnés ci-dessus pour la stimulation épi-rétinienne.

Si la prothèse est implantée en position sous-rétinienne, elle influence plutôt le potentiel électrique de cellules bipolaires. Dans ce cas, le signal de commande S délivré par l'unité de traitement 30 de la figure 1 peut être celui $V_{ON}^{exc}$, $V_{OFF}^{exc}$, $V_{ON}^{inh}$, $V_{OFF}^{inh}$ produit par un élément 56, 57 intervenant dans la modélisation des cellules bipolaires. De nouveau, le type de cellules bipolaires à stimuler et le schéma de modélisation correspondant peuvent être sélectionnés pour un patient donné en lui faisant suivre des tests psychophysiques.

La résolution spatiale des zones de pixel dans la prothèse 20 n'est pas nécessairement la même que celle des pixels dans le capteur DVS 10. Un ré-échantillonnage spatial du signal peut donc intervenir dans la transformation du signal d'entrée f en un signal de commande S. Dans le cas typique où la résolution est plus faible au niveau de la prothèse 20 qu'au niveau du capteur 10, le sous-échantillonnage spatial peut notamment intervenir lors de la dernière opération de filtrage spatial effectuée dans la transformation.

Le dispositif d'aide à la vision 20 peut être autre qu'une prothèse excitant électriquement des cellules du système visuel. Dans le cas d'une orthèse visuelle, le convertisseur peut correspondre à une matrice d'éléments photo-émetteurs (LED, MicroOLED, écran à cristaux liquides...) qui reprend les signaux issus des différents niveaux d'intégration du signal pour en produire une représentation visuelle.

L'utilisation d'orthèses commandées de cette manière peut notamment intervenir en liaison avec la thérapie génique qui est l'une des stratégies thérapeutiques pour les maladies dégénératives des photorécepteurs. Une modalité de la thérapie génique consiste à faire exprimer des canaux ioniques photosensibles ou des transporteurs photosensibles dans les cellules restantes de la rétine (photorécepteurs ayant perdu leur photosensibilité, cellules bipolaires, amacrines ou ganglionnaires). Cette modification génétique permet de 'créer' de nouveaux photorécepteurs qui peuvent être excités par la lumière. Cependant, leur sensibilité est faible comparée à celle des cônes et des bâtonnets. D'autre part, suivant le type cellulaire concerné, un traitement de l'information visuelle peut être effectué comme pour les prothèses utilisant les stimulations électriques. C'est pourquoi il est utile dans un tel cas d'utiliser un dispositif d'aide à la vision qui engendre une stimulation non plus électrique mais lumineuse et qui nécessite le même type de traitement.

Les modes de réalisation décrits ci-dessus sont des illustrations de la présente invention. Diverses modifications peuvent leur être apportées sans sortir du cadre de l'invention qui ressort des revendications annexées.

## Revendications

1. Dispositif de traitement de signal pour la commande d'un dispositif d'aide à la vision (20), comprenant
une entrée recevant un signal d'entrée (f) représentatif d'une scène à visualiser, le signal d'entrée comprenant, pour chaque pixel d'une matrice de pixels, une séquence de signal asynchrone basé sur événement obtenue en fonction de variations de lumière concernant le pixel dans la scène,
une sortie délivrant des signaux de commande (S) pour des zones de pixel respectives du dispositif d'aide à la vision, et
un circuit de traitement (30) configuré pour générer les signaux de commande en transformant le signal d'entrée spatialement au sein de la matrice de pixels et temporellement le long des séquences de signal,
dans lequel la transformation du signal d'entrée (f) effectuée par le circuit de traitement (30) pour générer les signaux de commande (S) comporte :

   - obtenir un premier signal ($V^{BC}$) résultant de deux opérations de filtrage spatial avec des noyaux de filtrage (50, 51) de tailles différentes, du calcul d'une différence entre les résultats des deux opérations de filtrage spatial et d'une opération (54, 55) de filtrage temporel de la différence; et

   - obtenir un second signal ( $V_{ON}^{exc}$ , $V_{ON}^{inh}$ , $V_{OFF}^{exc}$ , $V_{OFF}^{inh}$ ) de valeur nulle si le premier signal a une valeur d'un signe déterminé, et de même valeur absolue que le premier signal sinon.

2. Dispositif selon la revendication 1, dans lequel la séquence de signal asynchrone basé sur événement pour un pixel comporte une séquence d'impulsions positives ou négatives positionnées dans le temps en fonction des variations de lumière concernant le pixel.

3. Dispositif selon l'une des revendications 1 ou 2, dans lequel le dispositif d'aide à la vision comporte une prothèse visuelle (20) en position sous-rétinienne, et les signaux de commande (S) appliqués à la prothèse visuelle sont générés par le circuit de traitement (30) à partir dudit second signal.

4. Dispositif selon l'une des revendications 1 ou 2 dans lequel le dispositif d'aide à la vision comporte une matrice d'éléments photo-émetteurs et les signaux de commande (S) appliqués à cette matrice sont générés par le circuit de traitement (30) à partir dudit second signal.

5. Dispositif selon l'une des revendications 1 ou 2 dans lequel au moins un premier signal d'excitation et un premier signal d'inhibition sont obtenus avec des constantes de temps respectives ($\tau^{exc}$, $\tau^{inh}$) pour l'opération (54, 55) de filtrage temporel de la différence, et dans lequel au moins un second signal d'excitation ($V_{ON}^{exc}$, $V_{OFF}^{exc}$) et un second signal d'inhibition ($V_{ON}^{inh}$, $V_{OFF}^{inh}$) sont respectivement obtenus par le circuit de traitement (30) à partir des premiers signaux d'excitation et d'inhibition.

6. Dispositif selon la revendication 5, dans lequel la transformation du signal d'entrée (f) effectuée par le circuit de traitement (30) pour générer les signaux de commande (S) comporte en outre:

   - obtenir un troisième signal ($V^{GC}$) résultant d'une opération (64) de filtrage spatial de la différence entre le second signal d'excitation ($V_{ON}^{exc}$, $V_{OFF}^{exc}$) et une composante d'inhibition ($V^{AC}$) dérivée du second signal d'inhibition ($V_{ON}^{inh}$, $V_{OFF}^{inh}$) ; et

- lorsque le troisième signal pour une zone de pixel donnée du dispositif d'aide à la vision (20) dépasse une valeur de seuil déterminée ($\theta$), insérer une impulsion dans le signal de commande (S) destiné à ladite zone de pixel et remettre à zéro le troisième signal pour ladite zone de pixel.

**7.** Dispositif selon la revendication 6, dans lequel la dérivation de la composante d'inhibition ($V^{AC}$) à partir du second signal d'inhibition $\left(V_{ON}^{inh}, V_{OFF}^{inh}\right)$ comprend l'application (59) d'un retard prédéterminé (D) et une opération (60) de filtrage spatial.

**8.** Dispositif selon la revendication 7, dans lequel l'opération (60) de filtrage spatial dans la dérivation de la composante d'inhibition ($V^{AC}$) utilise un noyau de filtrage excentré.

**9.** Dispositif selon la revendication 5, dans lequel des seconds signaux d'excitation et d'inhibition $\left(V_{ON}^{exc}, V_{ON}^{inh}, V_{OFF}^{exc}, V_{OFF}^{inh}\right)$ ont obtenus par le circuit de traitement (30) d'une part pour un premier canal et d'autre part pour un second canal avec des opérations de filtrage temporel (54, 55) à constantes de temps respectives, et dans lequel la transformation du signal d'entrée (f) effectuée par le circuit de traitement (30) pour générer les signaux de commande (S) comporte en outre:

- obtenir un troisième signal ($V^{GC}$) résultant d'une opération (64) de filtrage spatial de la différence entre une combinaison linéaire des seconds signaux d'excitation $\left(V_{ON}^{exc}, V_{OFF}^{exc}\right)$ pour les premier et second canaux et une composante d'inhibition ($V^{AC}$) dérivée des seconds signaux d'inhibition $\left(V_{ON}^{inh}, V_{OFF}^{inh}\right)$ pour les premier et second canaux; et
- lorsque le troisième signal pour une zone de pixel donnée du dispositif d'aide à la vision (20) dépasse une valeur de seuil déterminée ($\theta$), insérer une impulsion dans le signal de commande (S) destiné à ladite zone de pixel et remettre à zéro le troisième signal pour ladite zone de pixel.

**10.** Dispositif selon la revendication 9, dans lequel la dérivation de la composante d'inhibition ($V^{AC}$) à partir des seconds signaux d'inhibition $\left(V_{ON}^{inh}, V_{OFF}^{inh}\right)$ comprend l'application de retards respectifs ($D_{ON}, D_{OFF}$) aux seconds signaux d'inhibition pour les premier et second canaux, une opération (60) de filtrage spatial des seconds signaux d'inhibition retardés et le calcul (68, 69) d'une combinaison linéaire des seconds signaux d'inhibition retardés et filtrés.

**11.** Dispositif selon l'une quelconque des revendications 6 à 10, dans lequel le dispositif d'aide à la vision comporte une prothèse visuelle (20) en position épi-rétinienne ou corticale ou sur le corps genouillé latéral, et les signaux de commande (S) appliqués à la prothèse visuelle sont générés par le circuit de traitement (30) à partir dudit troisième signal.

**12.** Dispositif selon l'une quelconque des revendications 6 à 10, dans lequel le dispositif d'aide à la vision comporte une matrice d'éléments photo-émetteurs (20) et les signaux de commande (S) appliqués à cette matrice sont générés par le circuit de traitement (30) à partir dudit troisième signal.

**Patentansprüche**

**1.** Signalverarbeitungsvorrichtung zur Steuerung einer Sehhilfe-Vorrichtung (20), aufweisend:

einen Eingang, der ein Eingangssignal (f) empfängt, das eine zu visualisierende Szene repräsentiert, wobei das Eingangssignal für jedes Pixel einer Pixelmatrix eine Sequenz ereignisbasierter Asynchronsignale aufweist, die als Funktion von Lichtänderungen, die das Pixel in der Szene betreffen, gewonnen wird, einen Ausgang, der Steuersignale (S) für jeweilige Pixelzonen der Sehhilfe-Vorrichtung ausgibt, und eine Verarbeitungsschaltung (30) zur Erzeugung der Steuersignale durch Transformieren des Eingangssignals räumlich in der Pixelmatrix und zeitlich entlang der Signalsequenzen,

wobei die von der Verarbeitungsschaltung (30) zur Erzeugung der Steuersignale (S) durchgeführte Transformation des Eingangssignals (f) aufweist:

- Gewinnen eines ersten Signals ($V^{BC}$), das sich aus zwei Raumfilteroperationen mit Filterkemen (50, 51) unterschiedlicher Größe, der Berechnung einer Differenz zwischen den Ergebnissen der zwei Raumfilteroperationen und einer Zeitfilteroperation (54, 55) der Differenz ergibt; und

- Gewinnen eines zweiten Signals ($V_{ON}^{exc}$, $V_{ON}^{inh}$, $V_{OFF}^{exc}$, $V_{OFF}^{inh}$) mit dem Wert Null, wenn das erste Signal einen Wert eines vorgegebenen Vorzeichens hat, und anderenfalls mit einem gleichen Absolutwert wie das erste Signal.

**2.** Vorrichtung nach Anspruch 1, in welcher die Sequenz ereignisbasierter Asynchronsignale für ein Pixel eine Sequenz positiver oder negativer, in der Zeit positionierter Pulse als Funktion der das Pixel betreffenden Lichtvariationen aufweist.

**3.** Vorrichtung nach einem der Ansprüche 1 oder 2, in welcher die Sehhilfe-Vorrichtung eine Sehprothese (20) in subretinaler Position aufweist und die an die Sehprothese angelegten Steuersignale (S) von der Verarbeitungsschaltung (30) ausgehend von dem zweiten Signal erzeugt werden.

**4.** Vorrichtung nach einem der Ansprüche 1 oder 2, in welcher die Sehhilfe-Vorrichtung eine Matrix lichtemittierender Elemente aufweist und die an diese Matrix angelegten Steuersignale (S) von der Verarbeitungsschaltung (30) ausgehend von dem zweiten Signal erzeugt werden.

**5.** Vorrichtung nach einem der Ansprüche 1 oder 2, in welcher mindestens ein erstes Erregungssignal und ein erstes Unterdrückungssignal mit jeweiligen Zeitkonstanten ($\tau^{exc}$, $\tau^{inh}$) für die Zeitfilteroperation (54, 55) der Differenz gewonnen werden und in welcher von der Verarbeitungsschaltung (30) ausgehend von dem ersten Erregungs- und Unterdrückungssignal mindestens ein zweites Erregungssignal ($V_{ON}^{exc}$, $V_{OFF}^{exc}$) bzw. ein zweites Unterdrückungssignal ($V_{ON}^{inh}$, $V_{OFF}^{inh}$) gewonnen werden.

**6.** Vorrichtung nach Anspruch 5, in welcher die von der Verarbeitungsschaltung (30) zur Erzeugung der Steuersignale (S) durchgeführte Transformation des Eingangssignals (f) ferner aufweist:

- Gewinnen eines dritten Signals ($V^{GC}$), das sich aus einer Raumfilteroperation der Differenz zwischen dem zweiten Erregungssignal ($V_{ON}^{exc}$, $V_{OFF}^{exc}$) und einer von dem zweiten Unterdrückungssignal ($V_{ON}^{inh}$, $V_{OFF}^{inh}$) hergeleiteten Unterdrückungskomponente ($V^{AC}$) ergibt; und

- wenn das dritte Signal für eine gegebene Pixelzone der Sehhilfe-Vorrichtung (20) einen vorgegebenen Schwellenwert ($\theta$) überschreitet, Einfügen eines Pulses in das für diese Pixelzone vorgesehene Steuersignal (S) und Zurücksetzen des dritten Signals für diese Pixelzone auf Null.

**7.** Vorrichtung nach Anspruch 6, in welcher die Herleitung der Unterdrückungskomponente ($V^{AC}$) ausgehend von dem zweiten Unterdrückungssignal ($V_{ON}^{inh}$, $V_{OFF}^{inh}$) die Anwendung (59) einer vorgegebenen Verzögerung (D) und eine Raumfilteroperation (60) aufweist.

**8.** Vorrichtung nach Anspruch 7, in welcher die Raumfilteroperation (60) in der Herleitung der Unterdrückungskomponente ($V^{AC}$) einen exzentrischen Filterkern verwendet.

**9.** Vorrichtung nach Anspruch 5, in welcher von der Verarbeitungsschaltung (30) zweite Erregungs- und Unterdrückungssignale ($V_{ON}^{exc}$, $V_{ON}^{inh}$, $V_{OFF}^{exc}$, $V_{OFF}^{inh}$) einerseits für einen ersten Kanal und andererseits für einen zweiten Kanal mit Zeitfilteroperationen (54, 55), die jeweilige Zeitkonstanten haben, gewonnen werden, und in welcher die von der Verarbeitungsschaltung (30) zur Erzeugung der Steuersignale (S) durchgeführte Transformation des Eingangssignals (f) ferner aufweist:

- Gewinnen eines dritten Signals ($V^{GC}$), das sich aus einer Raumfilteroperation (64) der Differenz zwischen einer linearen Kombination der zweiten Erregungssignale ($V_{ON}^{exc}$, $V_{OFF}^{exc}$) für den ersten und zweiten Kanal und einer von den zweiten Unterdrückungssignalen ($V_{ON}^{inh}$, $V_{OFF}^{inh}$) hergeleiteten Unterdrückungskomponente ($V^{AC}$) für den ersten und zweiten Kanal ergibt; und

- wenn das dritte Signal für eine gegebene Pixelzone der Sehhilfe-Vorrichtung (20) einen vorgegebenen Schwellenwert ($\theta$) überschreitet, Einfügen eines Pulses in das für diese Pixelzone vorgesehene Steuersignal (S) und Zurücksetzen des dritten Signals für diese Pixelzone auf Null.

**10.** Vorrichtung nach Anspruch 9, in welcher die Herleitung der Unterdrückungskomponente ($V^{AC}$) ausgehend von den zweiten Unterdrückungssignalen ($V_{ON}^{inh}$, $V_{OFF}^{inh}$) die Anwendung von jeweiligen Verzögerungen ($D_{ON}$, $D_{OFF}$) auf die zweiten Unterdrückungssignale für den ersten und zweiten Kanal, eine Raumfilteroperation (60) der zweiten verzögerten Unterdrückungssignale und die Berechnung (68, 69) einer linearen Kombination der verzögerten und gefilterten zweiten Unterdrückungssignale aufweist.

11. Vorrichtung nach einem der Ansprüche 6 bis 10, in welcher die Sehhilfe-Vorrichtung eine Sehprothese (20) in epiretinaler oder kortikaler Position oder an dem lateralen Kniekörper aufweist und die an die Sehprothese angelegten Steuersignale (S) von der Verarbeitungsschaltung (30) ausgehend von dem dritten Signal erzeugt werden.

12. Vorrichtung nach einem der Ansprüche 6 bis 10, in welcher die Sehhilfe-Vorrichtung eine Matrix (20) lichtemittierender Elemente aufweist und die an diese Matrix angelegten Steuersignale (S) von der Verarbeitungsschaltung (30) ausgehend von dem dritten Signal erzeugt werden.

**Claims**

1. A signal processing device for controlling a visual aid device (20), comprising:

an input receiving an input signal (f) representative of a scene to be viewed, the input signal comprising, for each pixel in a matrix of pixels, an event-based asynchronous signal sequence obtained as a function of variations of light relating to the pixel in the scene,
an output outputting control signals (S) for respective pixel zones of the visual aid device, and
a processing circuit (30) configured for generating the control signals by transforming the input signal spatially within the matrix of pixels and temporally along the signal sequences,

wherein the transforming of the input signal (f) performed by the processing circuit (30) for generating the control signals (S) comprises:

- obtaining a first signal ($V^{BC}$) resulting from two spatial filtering operations with filtering kernels (50, 51) of different sizes, calculation of a difference between the results of the two spatial filtering operations, and a temporal filtering operation (54, 55) on the difference; and

- obtaining a second signal ($V_{ON}^{exc}$, $V_{ON}^{inh}$, $V_{OFF}^{exc}$, $V_{OFF}^{inh}$) of zero value if the first signal has a value of a determined sign, and of same absolute value as the first signal otherwise.

2. The device of claim 1, wherein the event-based asynchronous signal sequence for a pixel comprises a sequence of positive or negative pulses temporally positioned as a function of the light variations relating to the pixel.

3. The device of any of claims 1 or 2, wherein the vision aid device comprises a subretinal visual prosthesis (20), and the control signals (S) applied to the visual prosthesis are generated by the processing circuit (30) from said second signal.

4. The device of any of claims 1 or 2, wherein the visual aid device comprises a matrix of light-emitting elements and the control signals (S) applied to said matrix are generated by the processing circuit (30) from said second signal.

5. The device of any of claims 1 or 2, wherein at least a first excitatory signal and a first inhibitory signal are obtained with respective time constants ($\tau^{exc}$, $\tau^{inh}$) for the temporal filtering operation (54, 55) on the difference, and wherein at least a second excitatory signal ($V_{ON}^{exc}$, $V_{OFF}^{exc}$) and a second inhibitory signal ($V_{ON}^{inh}$, $V_{OFF}^{inh}$) are respectively obtained by the processing circuit (30) from the first excitatory and inhibitory signals.

6. The device of claim 5, wherein the transforming of the input signal (f) performed by the processing circuit (30) to generate the control signals (S) further comprises:

- obtaining a third signal ($V^{GC}$) resulting from a spatial filtering operation (64) on the difference between the second excitatory signal ($V_{ON}^{exc}$, $V_{OFF}^{exc}$) and an inhibitory component ($V^{AC}$) derived from the second inhibitory signal ($V_{ON}^{inh}$, $V_{OFF}^{inh}$); and
- when the third signal for a given pixel zone of the visual aid device (20) exceeds a predetermined threshold value (0), inserting a pulse into the control signal (S) intended for said pixel zone and resetting the third signal for said pixel zone to zero.

7. The device of claim 6, wherein the derivation of the inhibitory component ($V^{AC}$) from the second inhibitory signal $\left(V_{ON}^{inh}, V_{OFF}^{inh}\right)$ comprises the application (59) of a predetermined delay (D) and a spatial filtering operation (60).

8. The device of claim 7, wherein the spatial filtering operation (60) in the derivation of the inhibitory component ($V^{AC}$) uses an off-center filtering kernel.

9. The device of claim 5, wherein the second excitatory and inhibitory signals $\left(V_{ON}^{exc}, V_{ON}^{inh}, V_{OFF}^{exc}, V_{OFF}^{inh}\right)$ are obtained by the processing circuit (30) for a first channel on the one hand and for a second channel on the other hand with temporal filtering operations (54, 55) at respective time constants, and wherein the transforming of the input signal (f) performed by the processing circuit (30) to generate the control signals (S) further comprises:

   - obtaining a third signal ($V^{GC}$) resulting from a spatial filtering operation (64) on the difference between a linear combination of the second excitatory signals $\left(V_{ON}^{exc}, V_{OFF}^{exc}\right)$ for the first and second channels and an inhibitory component ($V^{AC}$) derived from the second inhibitory signals $\left(V_{ON}^{inh}, V_{OFF}^{inh}\right)$ for the first and second channels; and
   - when the third signal for a given pixel zone of the visual aid device (20) exceeds a given threshold value (0), inserting a pulse into the control signal (S) for said pixel zone and resetting the third signal for said pixel zone to zero.

10. The device of claim 9, wherein the derivation of the inhibitory component ($V^{AC}$) from the second inhibitory signals $\left(V_{ON}^{inh}, V_{OFF}^{inh}\right)$ comprises the application of respective delays ($D_{ON}, D_{OFF}$) to the second inhibitory signals for the first and second channels, a spatial filtering operation (60) on the delayed second inhibitory signals, and calculation (68, 69) of a linear combination of delayed and filtered second inhibitory signals.

11. The device of any of claims 6 to 10, wherein the visual aid device comprises a visual prosthesis (20) in an epiretinal or cortical position or on the lateral geniculate body, and the control signals (S) applied to the visual prosthesis are generated by the processing circuit (30) from said third signal.

12. The device of any of claims 6 to 10, wherein the visual aid device comprises a matrix of light-emitting elements (20) and the control signals (S) applied to this matrix are generated by the processing circuit (30) from said third signal.

FIG. 1

FIG. 2A

FIG. 2B

FIG. 2C

FIG. 3A

FIG. 3B

FIG. 4

FIG. 5

$V^{PR} = f * h^{PR}$

$V^{HC} = V^{PR} * h^{HC}$

$V^{BC} = (V^{PR} - V^{HC}) * h^{BC}$
$V^{ON,OFF} = max(0, \pm V^{BC})$

$V^{AC} = h^{AC} * V^{ON,OFF}$

$V^{GC} = h^{GC} * (V^{ON,OFF} - \alpha \, V^{AC})$

FIG. 6

FIG. 7

FIG. 8

FIG. 9

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20100067825 A1
- FR 1053381

- US 20080135731 A1

**Littérature non-brevet citée dans la description**

- **T. GOLLISCH et al.** Rapid Neural Coding in the Retina with Relative Spike Latencies. *Science,* Février 2008, vol. 319, 1108-1111
- **ACOSTA-JIMÉNEZ et al.** AER Building Blocks for Multi-Layer Multi-Chip Neuromorphic Vision Systems. *Neural Information Processing Systems,* Décembre 2005, URL:http://www.ini.ethz.ch/~tobi/caviar/caviarPosterNIPS2005.pdf
- **R. SERRANO-GOTARREDONA et al.** AER Building Blocks for Multi-Layer Multi-Chip Neuromorphic Vision Systems. *Advances in neural information processing systems,* 2005, 18, URL:https://papers.nips.cc/paper/2889-aer-building-blocks-for-multi-layer-multi-chip-neuromorphic-vision-systems.pdf

- **P. LICHTSTEINER et al.** A $128\times128$ 120 dB 15 $\mu$s Latency Asynchronous Temporal Contrast Vision Sensor. *IEEE Journal of Solid-State Circuits,* Février 2008, vol. 43 (2), 566-576
- **B. ROSKA et al.** Parallel Processing in Retinal Ganglion Cells: How Integration of Space-Time Patterns of Excitation and Inhibition Form the Spiking Output. *Journal of Neurophysiology,* 2006, vol. 95 (6), 3810-3822